(19) Europäisches Patentamt
European Patent Office
Office européen des brevets

(11) **EP 4 249 905 B1**

(12) **EUROPEAN PATENT SPECIFICATION**

(45) Date of publication and mention
of the grant of the patent:
**24.12.2025 Bulletin 2025/52**

(21) Application number: **23162717.5**

(22) Date of filing: **17.03.2023**

(51) International Patent Classification (IPC):
**G01N 30/86** *(2006.01)*       **G01N 30/88** *(2006.01)*
**G01N 30/96** *(2006.01)*

(52) Cooperative Patent Classification (CPC):
**G01N 30/88; G01N 30/86; G01N 30/8675;**
G01N 30/8634; G01N 30/96; G01N 2030/8822

(54) **METHOD OF MEASURING HEMOGLOBIN F**

VERFAHREN ZUR MESSUNG VON HÄMOGLOBIN F

PROCÉDÉ DE MESURE DE L'HÉMOGLOBINE F

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **22.03.2022 JP 2022045897**

(43) Date of publication of application:
**27.09.2023 Bulletin 2023/39**

(73) Proprietor: **ARKRAY, Inc.
Kyoto-shi, Kyoto 601-8045 (JP)**

(72) Inventor: **ISHIKAWA, Kazuki
Kyoto, 602-0008 (JP)**

(74) Representative: **Dehns
10 Old Bailey
London EC4M 7NG (GB)**

(56) References cited:
**JP-A- 2001 228 133      JP-A- 2021 001 804**

**Description**

BACKGROUND

Technical Field

The present invention relates to a method of measuring hemoglobin F in a blood sample.

Related Art

Hemoglobin in blood samples can be measured by separation/fractionation methods such as high-performance liquid chromatography. Since a measured value of hemoglobin F (HbF), which is a type of hemoglobin, can be used as a basis for diagnosis of hemoglobinopathy and thalassemia, the value needs to be highly accurate. Examples of a method of measuring hemoglobin F using liquid chromatography include the method disclosed in Japanese Patent Application Laid-Open (JP-A) No. 2014-235023.

A chromatogram obtained by subjecting a blood sample to cation-exchange liquid chromatography shows, in the side with a higher rate of elution from the column relative to the HbF peak, a composite peak formed by overlapping peaks of hemoglobin A1a (HbA1a) and hemoglobin A1b (HbA1b), which are types of hemoglobin A1 (HbA1), which is a glycosylated product of hemoglobin A (HbA).

In view of this, an aspect of the present disclosure provides a technique that enables a more accurate measurement of HbF from a chromatogram obtained by subjecting a blood sample to liquid chromatography.

JP 2001-228133 A discloses a method of precisely measuring stabilized HbA1c using cation exchange liquid chromatography. The method sets the elution condition so that the resolution between most adjacent peaks of each peak of HbA1a, HbA1b and HbF is 0.8 or less.

JP 2021-001804 A discloses that when hemoglobin in blood is separated and analyzed by capillary electrophoresis, a fraction containing HbF is detected, and then a fraction containing chemically modified HbA0 and unstable A1c is detected. After that, in order, a fraction containing stable A1c, a fraction adjacent to the fraction containing HbA0, and a fraction containing HbA0 are detected.

SUMMARY

As in the process in which HbA is glycosylated to become HbA1c, it can be assumed that HbF may become modified HbF when it is modified by glycosylation or the like. Since the modified HbF is also a type of HbF, measurement of the peak value of the modified HbF is also required for more accurate measurement of HbF. In an experiment by the present inventors, the size of the composite peak was found to vary in accordance with the size of the HbF peak. From this observation result, it was assumed that the composite peak also includes a modified product of HbF. Since the HbA1a, HbA1b, and modified HbF included in the composite peak are similar to each other in terms of the charge and the size, it is difficult to separate the HbA1a, HbA1b, and modified HbF from each other by cation-exchange chromatography, and hence the peak value of the modified HbF cannot be accurately measured. As a result, it is impossible to measure the total amount of HbF (in other words, the sum of the unmodified HbF concentration and the modified HbF concentration) in the blood samples.

The present invention is defined in the appended claims. In one mode of the method of measuring HbF according to the present invention, a first correlation equation is preliminarily determined from a chromatogram obtained by subjecting, to liquid chromatography, a first blood sample group which is known to contain HbA1c, and whose content ratio of HbF in total hemoglobin is known to be less than a predetermined content ratio, wherein the first correlation equation is a correlation equation between an HbA 1c peak value and a composite peak value including an HbA1a peak and an HbA1b peak. A composite peak value obtained by applying an HbA1c peak value of a measurement target blood sample to the first correlation equation is subtracted from a composite peak value including an HbA1a peak and an HbA1b peak in the measurement target blood sample, to calculate a modified HbF peak value. The modified HbF peak value is added to an HbF peak value of the measurement target blood sample, to correct the HbF peak value.

According to the invention, HbF can be more accurately measured from a chromatogram obtained by subjecting a blood sample to liquid chromatography.

BRIEF DESCRIPTION OF THE DRAWINGS

Exemplary embodiments will be described in detail based on the following figures, wherein:

Fig. 1 schematically shows an example of a chromatogram of hemoglobin obtained by subjecting a blood sample to high-performance liquid chromatography;

Fig. 2 schematically shows a relationship between an HbF peak and a composite peak in a normal sample;

Fig. 3 schematically shows a relationship between an HbF peak and a composite peak in a high-HbF sample;

Fig. 4 is a chromatogram obtained by subjecting blood sample 1 to high-performance liquid chromatography;

Fig. 5 is a chromatogram obtained by subjecting blood sample 2 to high-performance liquid chromatography;

Fig. 6 is a chromatogram obtained by subjecting blood sample 3 to high-performance liquid chromatography;

Fig. 7 is a chromatogram obtained by subjecting blood sample 4 to high-performance liquid chromatography;

Fig. 8 is a scatter diagram illustrating a correlation between an HbA1c peak value and a composite peak value including an HbA1a peak and an HbA1b peak in a chromatogram obtained by subjecting a blood sample group of healthy individuals to high-performance liquid chromatography; and

Fig. 9 is a scatter diagram illustrating a correlation between an HbF peak value and a modified HbF peak value in a chromatogram obtained by subjecting a blood sample group that is known to include HbF, to high-performance liquid chromatography.

## DETAILED DESCRIPTION

Embodiments in the disclosure are described below with reference to drawings. The symbols shared among the diagrams represent identical portions even without any description. The "peak value" in the disclosure means the height or area of each peak found in a chromatogram, and the value may be either a relative value or an absolute value. The relative value may be the ratio to the total area of the chromatogram, may be the ratio to the total area of the peaks related to hemoglobin in the chromatogram, or may be the ratio to the area of a specific peak (such as an HbA0 peak).

(1) First Embodiment

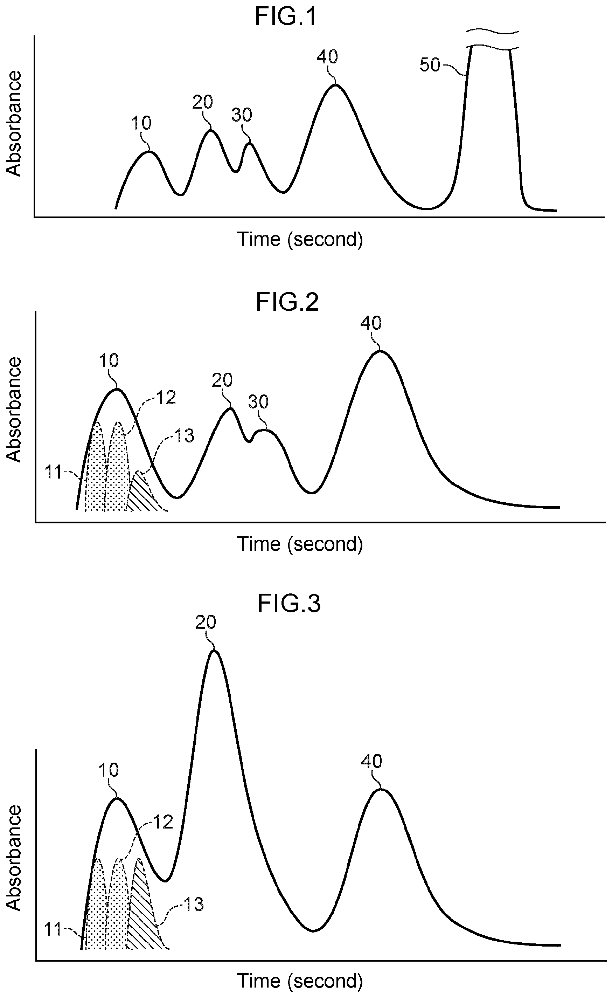

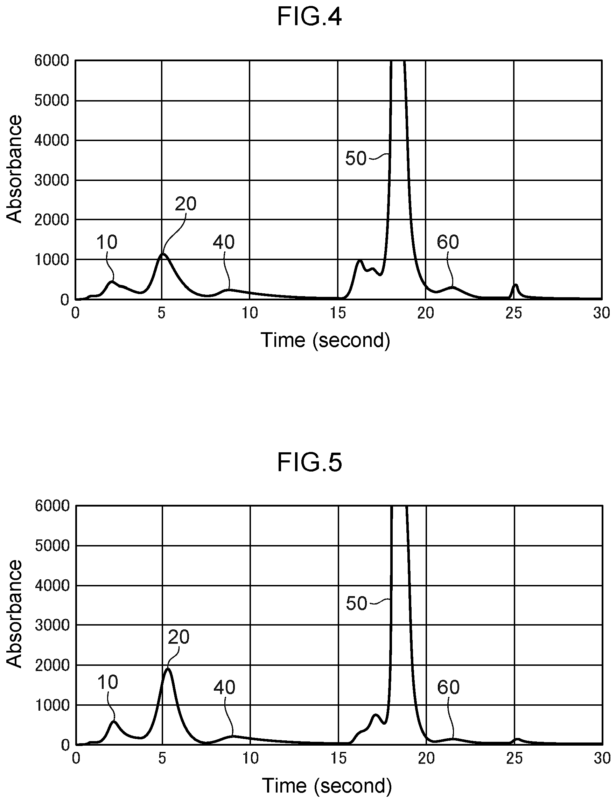

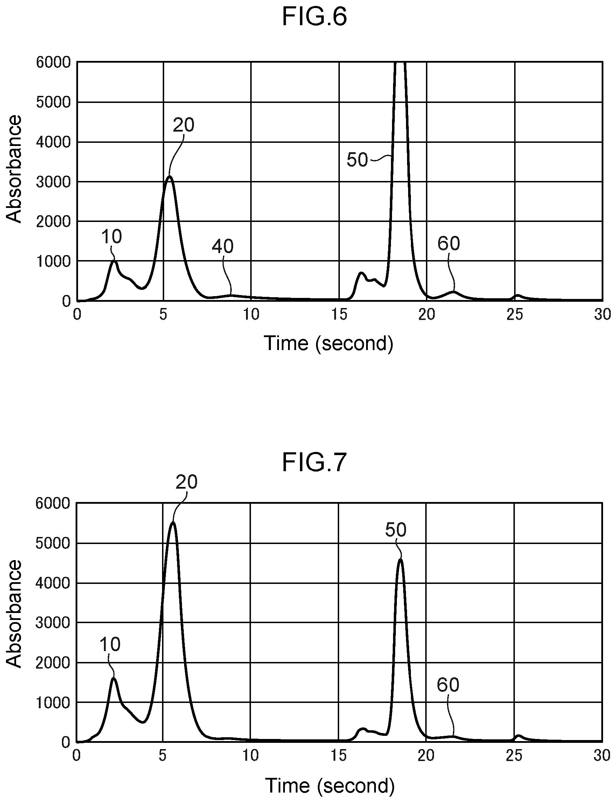

When a blood sample is subjected to cation-exchange liquid chromatography, a chromatogram of hemoglobin such as the one schematically represented in Fig. 1 can be obtained. In this chromatogram, a composite peak 10 including an HbA1a peak 11 and an HbA1b peak 12 (see Fig. 2 and Fig. 3), an HbF peak 20, an unstable HbA1c peak 30, an HbA1c peak 40, an HbA0 peak 50, and an HbA2 peak 60 (see Fig. 4 to Fig. 7) appear in the order of increasing rate of elution from the column.

Fig. 2 schematically shows a chromatogram of a normal blood sample having an HbF peak value of less than 1%, and Fig. 3 schematically shows a chromatogram of a blood sample of a high-HbF patient. As can be seen by comparison between these figures, which show HbA1c peaks 40 of almost the same size, the HbF peak 20 in the chromatogram of Fig. 3 is higher than the HbF peak 20 in the chromatogram of Fig. 2. In this case, the composite peak 10 in the chromatogram of Fig. 3 is also higher than the composite peak 10 in the chromatogram of Fig. 2.

Here, as shown in Fig. 2, the composite peak 10 includes an HbA1a peak 11 and an HbA1b peak 12 of HbA1, which is a glycosylated product of HbA. As shown in Fig. 3, an increase in the HbF peak 20 results in an increase in the composite peak 10. Since the composite peak 10 includes the HbA1a peak 11 and the HbA1b peak 12, which are glycosylated products, the increase in the composite peak 10 is thought to be due to an increase in a modified HbF peak 13, which is a modified product of HbF, as shown in Fig. 3. In contrast, the normal sample shown in Fig. 2 hardly includes the modified HbF peak 13, and most part of the composite peak 10 is thought to be formed by the HbA1a peak 11 and the HbA1b peak 12.

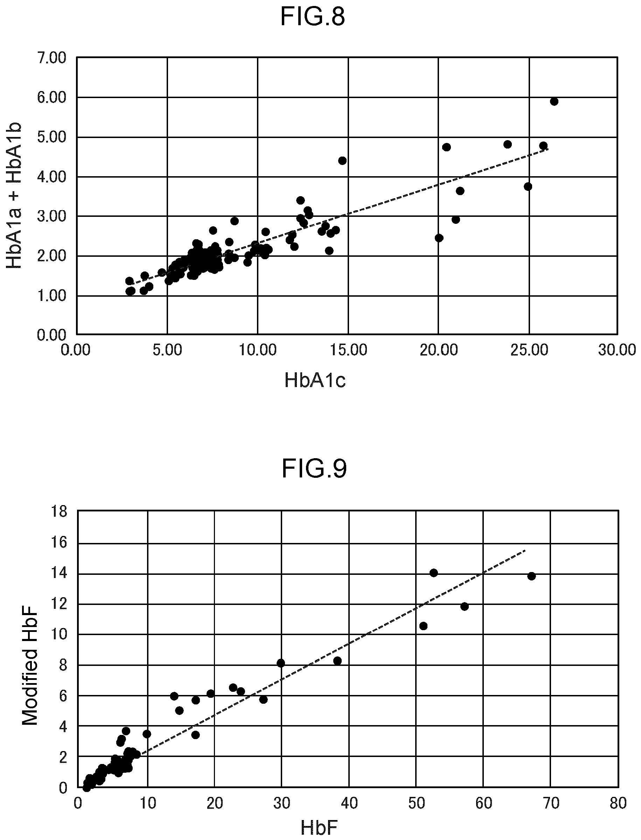

On the other hand, providing that, in both of a normal sample as shown in Fig. 2 and a high-HbF sample as shown in Fig. 3, the ratio of the sum of the HbA1a peak 11 value and the HbA1b peak 12 value to the HbA1c peak 40 is almost constant, if a correlation between the peak value of the HbA1c peak 40 and the peak value of the composite peak 10 (which is substantially the sum of the peak value of the HbA1a peak 11 and the peak value of the HbA1b peak 12) can be discovered, the correlation can be applied to the HbA1c peak 40 of a high-HbF sample to calculate the sum of the HbA1a peak 11 and the HbA1b peak 12 in the composite peak 10. The difference between the sum of the peak value of the HbA1a peak 11 and the peak value of the HbA1b peak 12, and the peak value of the composite peak 10, derives from the peak value of the modified HbF peak 13. It is thought that, by subtracting the calculated sum from the composite peak 10 in the chromatogram shown in Fig. 3, the modified HbF peak 13 can be calculated. As shown in Examples below (see Fig. 8), it was found, for a plurality of normal samples, that there is a high correlation between the HbA1c peak 40 and the composite peak 10.

Based on this correlation, in the method of measuring HbF of a first embodiment of the disclosure, a first correlation equation is preliminarily determined from a chromatogram obtained by subjecting, to liquid chromatography, a first blood sample group which is known to contain HbA1c but not to contain HbF, wherein the first correlation equation is a correlation equation between an HbA1c peak value and a composite peak value including an HbA1a peak and an HbA1b peak. A composite peak value obtained by applying, to the first correlation equation, an HbA1c peak value of a chromatogram obtained by subjecting a measurement target blood sample to liquid chromatography is subtracted from a composite peak value including an HbA1a peak and an HbA1b peak of the measurement target blood sample, to calculate a modified HbF peak value. The modified HbF peak value is added to an HbF peak value of the measurement target blood sample, to correct the HbF peak value. Since the HbF peak value correlates with the concentration of HbF contained in the measurement target blood sample, it is possible to determine the concentration of HbF contained in the measurement target blood sample, the ratio of the amount of HbF relative to total hemoglobin, and the like based on the HbF peak value.

More specifically, for a plurality of blood samples which are known to contain HbA1c, and whose content ratios of HbF in total hemoglobin are known to be less than a predetermined content ratio, a first correlation equation is determined from a correlation between the HbA1c peak value and the composite peak value (which is substantially the sum of the HbA1a peak value and the HbA1b peak value). Note that the "predetermined content ratio" herein may be an HbF peak value of not more than a normal value (for example, 5%, preferably 3%, more preferably 1% relative to the peak value of total hemoglobin). The "plurality of blood samples whose content ratios of HbF in total hemoglobin are known to be less than a predetermined content ratio" may be obtained as blood samples which show HbF peak values of less than the predetermined content ratio as obtained by separation analysis of hemoglobin contained in the blood samples by liquid chromatography. Alternatively, for example, the plurality of blood samples may be obtained as blood samples whose hemoglobin F contents have been shown to be less than a predetermined content ratio by analysis using a measurement principle other than liquid chromatography (for example, capillary electrophoresis). The first correlation equation is as shown in Formula (1) below, wherein the HbA1c peak value is variable x, and the composite peak value is variable y.

$$y = a_1x + b_1 \cdots (1)$$

x: HbA1c peak value (variable)
y: composite peak value (variable)
$a_1$: slope (constant)
$b_1$: intercept (constant)

The HbA1c peak value is then determined from the chromatogram obtained from the measurement target blood sample, and the value is assigned to variable x of Formula (1) above, and the composite peak value is assumed as the calculated variable y. The calculated estimated value of the composite peak is subtracted from the composite peak value obtained from the composite peak 10 that appears in the chromatogram (i.e., the composite peak value measured using the composite peak 10 that appears in the chromatogram), to calculate the value of the modified HbF peak included in the composite peak 10. Then, by adding the calculated modified HbF peak value to the peak value of the HbF peak 20 obtained from the chromatogram, the HbF peak value is corrected to assume the true HbF peak value in the measurement target blood sample. The modified HbF peak value is as shown in the following Formula (2) below. When the modified HbF peak value is w, and the HbF peak value is variable z, the true HbF peak value (v) is as shown in the following Formula (3) below.

$$w = y - (a_1x + b_1) \cdots (2)$$

$$v = z + w = z + (y - (a_1x + b_1)) \cdots (3)$$

v: true HbF peak value (variable)
w: modified HbF peak value (variable)
x: HbA1c peak value (variable)
y: composite peak value (variable)
z: HbF peak value (variable)
$a_1$: slope (constant)
$b_1$: intercept (constant)

(2) Second Embodiment

The first embodiment shown above is dependent on the presence of HbA1c in the measurement target blood sample, and it is impossible to apply the HbA1c peak value to the first correlation equation in cases where the measurement target blood sample does not contain HbA1c, or where HbA1c is not detected in the measurement target blood sample.

In view of this, in the method of measuring HbF of a second embodiment, a first correlation equation is preliminarily determined as described in the first embodiment. Further, from a chromatogram obtained by subjecting a second blood sample group which is known to contain HbA1c and HbF to liquid chromatography, the HbA1c peak value is applied to the first correlation equation, to obtain a composite peak value. The composite peak value is subtracted from a composite peak value including an HbA1a peak and an HbA1b peak in the second blood sample group, to obtain an estimated modified HbF peak value of the second blood sample group. A second correlation equation, which is a correlation equation between an HbF peak value and the estimated modified HbF peak value in the second blood sample group, is preliminarily determined. The HbF peak value of a chromatogram obtained by subjecting the measurement target blood sample to liquid chromatography is applied to the second correlation equation, to calculate the modified HbF peak value. The modified HbF peak value is then added to the HbF peak value of the measurement target blood sample, to correct the HbF peak value.

More specifically, first, as described above in the first embodiment, the first correlation equation of Formula (1) shown above is determined.

Subsequently, for chromatograms of a plurality of blood samples which are known to contain both HbA1c and HbF, the HbA1c peak value is determined. The value is then assigned to variable x of Formula (1) shown above. Then, the composite peak value is assumed as the calculated variable y. The calculated composite peak value is subtracted from the composite peak value obtained from the composite peak 10 that appears in the chromatogram (i.e., the composite peak value measured using the composite peak 10 that appears in the chromatogram), to calculate the value of the modified HbF peak included in the composite peak 10. On the other hand, from the same chromatogram, the HbF peak value is obtained, and the second correlation equation is determined from a correlation between the HbF peak value and the calculated modified HbF peak value. The second correlation equation is as shown in Formula (4) below, wherein the HbF peak value is variable z, and the modified HbF peak value is variable w.

$$w = a_2 z + b_2 \cdots (4)$$

w: modified HbF peak value (variable)
z: HbF peak value (variable)
$a_2$: slope (constant)
$b_2$: intercept (constant)

The HbF peak value is then determined from a chromatogram obtained from the measurement target blood sample, and the value is assigned to variable z of Formula (4) shown above. Then, the modified HbF peak value is calculated as variable w. By adding the calculated modified HbF peak value to the HbF peak value obtained from the chromatogram, the HbF peak value is corrected to assume the true HbF peak value in the measurement target blood sample. The true HbF peak value in the measurement target blood sample (v) is as shown in the following Formula (5).

$$v = z + w = z + (a_2 z + b_2) \cdots (5)$$

v: true HbF peak value (variable)
w: modified HbF peak value (variable)
z: HbF peak value (variable)
$a_2$: slope (constant)
$b_2$: intercept (constant)

Note that, instead of the second correlation equation shown above, a third correlation equation may be determined from a correlation between the HbF peak value and the corrected HbF peak value (i.e., the sum of the HbF peak value and the modified HbF peak value). The third correlation equation is as shown in the following Formula (6), wherein the HbF peak value is variable z, and the corrected HbF peak value as the true HbF peak value is v.

$$v = a_3 z + b_3 \cdots (6)$$

v: true HbF peak value (variable)
z: HbF peak value (variable)
$a_3$: slope (constant)
$b_3$: intercept (constant)

The HbF peak value is then determined from a chromatogram obtained from the measurement target blood sample, and the value is assigned to variable z of Formula (5) or Formula (6) shown above. The corrected HbF peak value, that is, the true HbF peak value in the measurement target blood sample, is assumed as the calculated variable v.

(3) Third Embodiment

The first embodiment is suitable for blood samples containing HbA1c. The second embodiment is suitable for blood samples not containing HbA1c. Therefore, it is desirable to employ the measurement method of the first embodiment or the measurement method of the second embodiment depending on whether HbA1c is included in the chromatogram.

In view of this, in the method of measuring HbF of a third embodiment, the first correlation equation is preliminarily determined as described in the first embodiment, and the second correlation equation is preliminarily determined as described in the second embodiment. Then, in cases where the target chromatogram obtained by subjecting the measurement target blood sample to liquid chromatography has the HbA1c peak, the HbF peak value of the measurement target blood sample is corrected as described in the first embodiment. On the other hand, in cases where the target chromatogram obtained by subjecting the measurement target blood sample to liquid chromatography does not have the HbA1c peak, the HbF peak value of the measurement target blood sample is corrected as described in the second embodiment.

EXAMPLES

(1) Liquid Chromatography Apparatus

A commercially available cation-exchange chromatography column packed with a hydrophilic polymer containing a methacrylate copolymer was connected to a commercially available high-performance liquid chromatography apparatus. To a predetermined position of the downstream channel of the cation-exchange chromatography column, an optical detector (more specifically, an absorption spectrometer) that detects the concentration of hemoglobin passing through the channel was attached.

(2) Eluent

Three types of eluents were prepared as aqueous solutions having the compositions shown in Table 1 below.

[Table 1]

| Material | Concentration (mass%) | | |
|---|---|---|---|
| | Eluent A | Eluent B | Eluent C |
| Sodium dihydrogen phosphate dihydrate | 1.44 | 0.02 | 0.12 |
| Disodium hydrogen phosphate | 0.16 | 0.50 | 0.33 |

The pH of Eluent A was adjusted to 5.08; the pH of Eluent B was adjusted to 8.0; and the pH of Eluent C was adjusted to 6.82. Regarding the elution strength of these eluents for hemoglobin, Eluent A had the lowest strength, and Eluent B had the highest strength.

(3) Chromatogram

Eluent A was passed through the liquid chromatography apparatus of (1), to equilibrate the column. Thereafter, a hemolyzed blood sample was introduced into the column. Thereafter, Eluent A was passed through the column for 13 seconds, to elute HbA1a, HbA1b, HbF, and HbA1c. Subsequently, a mixed solution of Eluent A and Eluent C at 1:9 was passed through the column for 5 seconds, to elute HbA0. Subsequently, Eluent C was passed through the column for 5 seconds, to elute HbA2. Thereafter, Eluent B was passed through the column for 2 seconds to elute the entire hemoglobin remaining in the column, and then Eluent A was passed through the column for 5 seconds. During the elution, a chromatogram was prepared based on the absorbance obtained by the optical detector at a detection wavelength of 420 nm.

Examples of the obtained chromatogram are shown in Fig. 4 to Fig. 7. As is evident from these figures, as the area of the HbF peak 20 (HbF peak value) increased, the area of the composite peak 10 (composite peak value) increased. Thus, the composite peak 10, which is known to include the HbA1a peak 11 and the HbA1b peak 12 (see Fig. 3), was strongly assumed to include the modified HbF peak value 13 (see Fig. 3).

(4) First Correlation Equation

In order to determine the first correlation equation, blood samples of 66 healthy individuals (blood samples showing HbF peak values of less than 1% relative to the peak values of total hemoglobin) were provided. A chromatogram was obtained from each of these blood samples, and the HbA1c peak value and the composite peak value were measured for the chromatogram. As a result, it was found that there is a correlation as shown in the scatter diagram of Fig. 8. The abscissa of the graph represents the HbA1c peak value, and the ordinate represents the composite peak value (that is, the sum of the HbA1a peak value and the HbA1b peak value). The first correlation equation, represented by the dotted line in Fig. 8, was as shown in Formula (7) below, wherein the HbA1c peak value is variable x, and the composite peak value is variable y. The correlation coefficient (r) of the first correlation equation was 0.8951.

$$y = 0.1475x + 0.8048 \cdots (7)$$

x: HbA1c peak value (variable)
y: composite peak value (variable)

It can be assumed that the presence of the correlation between the composite peak 10 including HbA1a and HbA1b, and the HbA1c peak 40, may be due to the fact that the ratios of HbA1a, HbA1b, and HbA1c are almost constant since all of HbA1a, HbA1b, and HbA1c are glycosylated products of HbA0 and are produced as a result of glycosylation of HbA0.

The HbA1c peak value is then measured from the chromatogram obtained from the measurement target blood sample, and the value is assigned as variable x to Formula (7). As variable y, the sum of the HbA1a peak value and the HbA1b peak value (hereinafter referred to as "AB value") is calculated. The difference between the composite peak value measured from the chromatogram and the AB value derives from the modified HbF peak value. Thus, the AB value is subtracted from the composite peak value measured from the chromatogram, to calculate the modified HbF peak value. Then, the value obtained by adding the calculated modified HbF peak value to the HbF peak value measured from the chromatogram is assumed to be the true HbF peak value of the measurement target blood sample.

(2) Second Correlation Equation

In order to determine the second correlation equation, blood samples of 66 healthy individuals (blood samples showing HbF peak values of less than 1% relative to the peak values of total hemoglobin) and blood samples of 48 high-HbF patients were provided. A chromatogram was obtained from each of these blood samples. The HbA1c peak value was measured from the chromatogram, and the value was assigned as variable x to Formula (7), to calculate the AB value as variable y. The AB value was then subtracted from the composite peak value measured from the chromatogram, to calculate the modified HbF peak value. As a result, it was found that, between the HbF peak value measured from the chromatogram and the calculated modified HbF peak value, there is a correlation as shown in the scatter diagram of Fig. 9. The abscissa of the graph represents the HbF peak value, and the ordinate represents the modified HbF peak value. The second correlation equation, represented by the dotted line in Fig. 9, was as shown in Formula (8) below, wherein the HbF peak value is variable z, and the modified HbF peak value is variable w. The correlation coefficient (r) of the second correlation equation was 0.9697.

$$w = 0.2323z \cdots (8)$$

From a chromatogram obtained from the measurement target blood sample, the HbF peak value is measured, and the value is assigned as variable z to Formula (8), to calculate the modified HbF peak value as variable w. The value obtained by adding the calculated modified HbF peak value to the HbF peak value measured from the chromatogram is assumed to be the true HbF peak value of the measurement target blood sample.

Industrial Applicability

The invention is applicable to measurement of HbF in a blood sample using liquid chromatography.

**Claims**

1. A method of measuring hemoglobin F for a measurement target blood sample, the method comprising:

preliminarily determining a first correlation equation from a chromatogram obtained by subjecting a first blood sample group to liquid chromatography, the first blood sample group being known to contain hemoglobin A1c, and being known to contain less hemoglobin F than a predetermined content ratio relative to total hemoglobin, and the first correlation equation being a correlation equation between a hemoglobin A1c peak (40) value and a composite peak value including a hemoglobin A1a peak (11) and a hemoglobin A1b peak (12);

calculating a modified hemoglobin F peak value by subtracting a composite peak value obtained by applying, to the first correlation equation, a hemoglobin A1c peak (40) value of a chromatogram obtained by subjecting a measurement target blood sample to liquid chromatography, from a composite peak value including a hemoglobin A1a peak (11) and a hemoglobin A1b peak (12) in the measurement target blood sample; and

correcting a hemoglobin F peak value by adding the modified hemoglobin F peak value to the hemoglobin F peak value of the measurement target blood sample.

2. A method of measuring hemoglobin F for a measurement target blood sample, the method comprising:

preliminarily determining a first correlation equation from a chromatogram obtained by subjecting a first blood sample group to liquid chromatography, the first blood sample group being known to contain hemoglobin A1c, and being known to contain less hemoglobin F than a predetermined content ratio relative to total hemoglobin, and the first correlation equation being a correlation equation between a hemoglobin A1c peak (40) value and a composite peak value including a hemoglobin A1a peak (11) and a hemoglobin A1b peak (12);

obtaining a composite peak value by applying a hemoglobin A1c peak (40) value to the first correlation equation, the hemoglobin A1c peak (40) value being obtained from a chromatogram obtained by subjecting a second blood sample group known to contain hemoglobin A1c and hemoglobin F to liquid chromatography;

obtaining an estimated modified hemoglobin F peak value of the second blood sample group by subtracting the composite peak value from a composite peak value including a hemoglobin A1a peak (11) and a hemoglobin A1b peak (12) in the second blood sample group;

preliminarily determining a second correlation equation using a hemoglobin F peak value and the estimated modified hemoglobin F peak value of the second blood sample group, the second correlation equation being a correlation equation between a hemoglobin F peak value and a modified hemoglobin F peak value;

calculating a modified hemoglobin F peak value by applying a hemoglobin F peak value of a chromatogram obtained by subjecting a measurement target blood sample to liquid chromatography, to the second correlation equation; and

correcting a hemoglobin F peak value by adding the modified hemoglobin F peak value to the hemoglobin F peak value of the measurement target blood sample.

3. A method of measuring hemoglobin F for a measurement target blood sample, the method comprising:

preliminarily determining a first correlation equation from a chromatogram obtained by subjecting a first blood sample group to liquid chromatography, the first blood sample group being known to contain hemoglobin A1c, and being known to contain less hemoglobin F than a predetermined content ratio relative to total hemoglobin, and the first correlation equation being a correlation equation between a hemoglobin A1c peak (40) value and a composite peak value including a hemoglobin A1a peak (11) and a hemoglobin A1b peak (12);

obtaining a composite peak value by applying a hemoglobin A1c peak (40) value to the first correlation equation, the hemoglobin A1c peak (40) value being obtained from a chromatogram obtained by subjecting a second blood sample group known to contain hemoglobin A1c and hemoglobin F to liquid chromatography;

obtaining an estimated modified hemoglobin F peak value of the second blood sample group by subtracting the composite peak value from a composite peak value including a hemoglobin A1a peak (11) and a hemoglobin A1b peak (12) in the second blood sample group; and

preliminarily determining a second correlation equation using a hemoglobin F peak value and the estimated modified hemoglobin F peak value of the second blood sample group, the second correlation equation being a correlation equation between a hemoglobin F peak value and a modified hemoglobin F peak value;

wherein, in a case in which a chromatogram obtained by subjecting a measurement target blood sample to liquid chromatography includes a hemoglobin A1c peak (40), the method further comprises:

calculating a modified hemoglobin F peak value by subtracting a composite peak value obtained by applying a hemoglobin A1c peak (40) value of the measurement target blood sample to the first correlation equation, from a composite peak value including a hemoglobin A1a peak (11) and a hemoglobin A1b peak (11) of the measurement target blood sample; and

correcting a hemoglobin F peak value by adding the modified hemoglobin F peak value to the hemoglobin F peak value of the measurement target blood sample, or

wherein, in a case in which a chromatogram obtained by subjecting a measurement target blood sample to liquid chromatography does not include a hemoglobin A1c peak (40), the method further comprises:

calculating a modified hemoglobin F peak value by applying a hemoglobin F peak value of the measurement target blood sample to the second correlation equation; and
correcting a hemoglobin F peak value by adding the modified hemoglobin F peak value to the hemoglobin F peak value of the measurement target blood sample.

4. The method of measuring hemoglobin F for the measurement target blood sample according to claim 2 or claim 3, wherein:

a third correlation equation that is a correlation equation between a hemoglobin F peak value and a sum of the hemoglobin F peak value and the estimated modified hemoglobin F peak value in the second blood sample group is preliminarily determined instead of the second correlation equation; and
a hemoglobin F peak value of a chromatogram obtained by subjecting the measurement target blood sample to liquid chromatography is applied to the third correlation equation instead of the second correlation equation, to correct the hemoglobin F peak value.

5. The method of measuring hemoglobin F for the measurement target blood sample according to any one of claim 1 to claim 4, wherein the liquid chromatography is cation-exchange chromatography.


**Patentansprüche**

1. Verfahren zur Messung von Hämoglobin F für eine Messzielblutprobe, wobei das Verfahren umfasst:

vorläufiges Bestimmen einer ersten Korrelationsgleichung aus einem Chromatogramm, das erhalten wird, indem eine erste Blutprobengruppe einer Flüssigchromatographie unterzogen wird, wobei von der ersten Blutprobengruppe bekannt ist, dass sie Hämoglobin A1c enthält, und bekannt ist, dass sie weniger Hämoglobin F als ein vorbestimmter Gehaltsanteil in Bezug auf Gesamthämoglobin enthält, und wobei die erste Korrelationsgleichung eine Korrelationsgleichung zwischen einem Spitzenwert von Hämoglobin A1c (40) und einem zusammengesetzten Spitzenwert ist, der eine Spitze von Hämoglobin A1a (11) und eine Spitze von Hämoglobin A1b (12) einschließt;
Berechnen eines modifizierten Spitzenwerts von Hämoglobin F, indem ein zusammengesetzter Spitzenwert, der erhalten wird, indem auf die erste Korrelationsgleichung ein Spitzenwert von Hämoglobin A1c (40) eines Chromatogramms angewendet wird, das erhalten wird, indem eine Messzielblutprobe einer Flüssigchromatographie unterzogen wird, von einem zusammengesetzten Spitzenwert, der eine Spitze von Hämoglobin A1a (11) und eine Spitze von Hämoglobin A1b (12) einschließt, in der Messzielblutprobe abgezogen wird; und
Korrigieren eines Spitzenwerts von Hämoglobin F durch Addieren des modifizierten Spitzenwerts von Hämoglobin F zum Spitzenwert von Hämoglobin F der Messzielblutprobe.

2. Verfahren zur Messung von Hämoglobin F für eine Messzielblutprobe, wobei das Verfahren umfasst:

vorläufiges Bestimmen einer ersten Korrelationsgleichung aus einem Chromatogramm, das erhalten wird, indem eine erste Blutprobengruppe einer Flüssigchromatographie unterzogen wird, wobei von der ersten Blutprobengruppe bekannt ist, dass sie Hämoglobin A1c enthält, und bekannt ist, dass sie weniger Hämoglobin F als ein vorbestimmter Gehaltsanteil in Bezug auf Gesamthämoglobin enthält, und wobei die erste Korrelationsgleichung eine Korrelationsgleichung zwischen einem Spitzenwert von Hämoglobin A1c (40) und einem zusammengesetzten Spitzenwert ist, der eine Spitze von Hämoglobin A1a (11) und eine Spitze von Hämoglobin A1b (12) einschließt;
Erhalten eines zusammengesetzten Spitzenwerts durch Anwenden eines Spitzenwerts von Hämoglobin A1c (40) auf die erste Korrelationsgleichung, wobei der Spitzenwert von Hämoglobin A1c (40) aus einem Chromatogramm erhalten wird, das erhalten wird, indem eine zweite Blutprobengruppe, von der bekannt ist, dass sie Hämoglobin A1c und Hämoglobin F enthält, einer Flüssigchromatographie unterzogen wird;
Erhalten eines geschätzten modifizierten Spitzenwerts von Hämoglobin F der zweiten Blutprobengruppe, indem der zusammengesetzte Spitzenwert von einem zusammengesetzten Spitzenwert, der eine Spitze von Hämoglobin A1a (11) und eine Spitze von Hämoglobin A1b (12) einschließt, in der zweiten Blutprobengruppe abgezogen wird; vorläufiges Bestimmen einer zweiten Korrelationsgleichung unter Verwendung eines Spitzenwerts von Hämoglobin F und des geschätzten modifizierten Spitzenwerts von Hämoglobin F der zweiten Blutprobengruppe, wobei die zweite Korrelationsgleichung eine Korrelationsgleichung zwischen einem Spitzenwert von Hämoglobin F und einem modifizierten Spitzenwert von Hämoglobin F ist;

Berechnen eines modifizierten Spitzenwerts von Hämoglobin F durch Anwenden eines Spitzenwerts von Hämoglobin F eines Chromatogramms, das erhalten wird, indem eine Messzielblutprobe einer Flüssigchromatographie unterzogen wird, auf die zweite Korrelationsgleichung; und

Korrigieren eines Spitzenwerts von Hämoglobin F durch Addieren des modifizierten Spitzenwerts von Hämoglobin F zum Spitzenwert von Hämoglobin F der Messzielblutprobe.

3. Verfahren zur Messung von Hämoglobin F für eine Messzielblutprobe, wobei das Verfahren umfasst:

vorläufiges Bestimmen einer ersten Korrelationsgleichung aus einem Chromatogramm, das erhalten wird, indem eine erste Blutprobengruppe einer Flüssigchromatographie unterzogen wird, wobei von der ersten Blutprobengruppe bekannt ist, dass sie Hämoglobin A1c enthält, und bekannt ist, dass sie weniger Hämoglobin F als ein vorbestimmter Gehaltsanteil in Bezug auf Gesamthämoglobin enthält, und wobei die erste Korrelationsgleichung eine Korrelationsgleichung zwischen einem Spitzenwert von Hämoglobin A1c (40) und einem zusammengesetzten Spitzenwert ist, der eine Spitze von Hämoglobin A1a (11) und eine Spitze von Hämoglobin A1b (12) einschließt;

Erhalten eines zusammengesetzten Spitzenwerts durch Anwenden eines Spitzenwerts von Hämoglobin A1c (40) auf die erste Korrelationsgleichung, wobei der Spitzenwert von Hämoglobin A1c (40) aus einem Chromatogramm erhalten wird, das erhalten wird, indem eine zweite Blutprobengruppe, von der bekannt ist, dass sie Hämoglobin A1c und Hämoglobin F enthält, einer Flüssigchromatographie unterzogen wird;

Erhalten eines geschätzten modifizierten Spitzenwerts von Hämoglobin F der zweiten Blutprobengruppe, indem der zusammengesetzte Spitzenwert von einem zusammengesetzten Spitzenwert, der eine Spitze von Hämoglobin A1a (11) und eine Spitze von Hämoglobin A1b (12) einschließt, in der zweiten Blutprobengruppe abgezogen wird; und

vorläufiges Bestimmen einer zweiten Korrelationsgleichung unter Verwendung eines Spitzenwerts von Hämoglobin F und des geschätzten modifizierten Spitzenwerts von Hämoglobin F der zweiten Blutprobengruppe, wobei die zweite Korrelationsgleichung eine Korrelationsgleichung zwischen einem Spitzenwert von Hämoglobin F und einem modifizierten Spitzenwert von Hämoglobin F ist;

wobei, in einem Fall, bei dem ein Chromatogramm, das erhalten wird, indem eine Messzielblutprobe einer Flüssigchromatographie unterzogen wird, eine Spitze von Hämoglobin A1c (40) einschließt, das Verfahren weiter umfasst:

Berechnen eines modifizierten Spitzenwerts von Hämoglobin **F,** indem ein zusammengesetzter Spitzenwert, der erhalten wird, indem ein Spitzenwert von Hämoglobin A1c (40) der Messzielblutprobe auf die erste Korrelationsgleichung angewendet wird, von einem zusammengesetzten Spitzenwert, der eine Spitze von Hämoglobin A1a (11) und eine Spitze von Hämoglobin A1b (11) einschließt, der Messzielblutprobe abgezogen wird; und

Korrigieren eines Spitzenwerts von Hämoglobin F durch Addieren des modifizierten Spitzenwerts von Hämoglobin F zum Spitzenwert von Hämoglobin F der Messzielblutprobe, oder

wobei, in einem Fall, bei dem ein Chromatogramm, das erhalten wird, indem eine Messzielblutprobe einer Flüssigchromatographie unterzogen wird, keine Spitze von Hämoglobin A1c (40) einschließt, das Verfahren weiter umfasst:

Berechnen eines modifizierten Spitzenwerts von Hämoglobin F durch Anwenden eines Spitzenwerts von Hämoglobin F der Messzielblutprobe auf die zweite Korrelationsgleichung; und

Korrigieren eines Spitzenwerts von Hämoglobin F durch Addieren des modifizierten Spitzenwerts von Hämoglobin F zum Spitzenwert von Hämoglobin F der Messzielblutprobe.

4. Verfahren zur Messung von Hämoglobin F für die Messzielblutprobe nach Anspruch 2 oder Anspruch 3, wobei:

eine dritte Korrelationsgleichung, die eine Korrelationsgleichung zwischen einem Spitzenwert von Hämoglobin F und einer Summe aus dem Spitzenwert von Hämoglobin F und dem geschätzten modifizierten Spitzenwert von Hämoglobin F in der zweiten Blutprobengruppe ist, anstelle der zweiten Korrelationsgleichung vorläufig bestimmt wird; und

ein Spitzenwert von Hämoglobin F eines Chromatogramms, das erhalten wird, indem die Messzielblutprobe einer Flüssigchromatographie unterzogen wird, auf die dritte Korrelationsgleichung anstelle der zweiten Korrelationsgleichung angewendet wird, um den Spitzenwert von Hämoglobin F zu korrigieren.

5. Verfahren zur Messung von Hämoglobin F für die Messzielblutprobe nach einem von Anspruch 1 bis Anspruch 4, wobei die Flüssigchromatographie eine Kationenaustausch-Chromatographie ist.

**Revendications**

1. Procédé de mesure de l'hémoglobine F pour un échantillon de sang cible de mesure, le procédé comprenant :

la détermination préliminaire d'une première équation de corrélation à partir d'un chromatogramme obtenu en soumettant un premier groupe d'échantillons de sang à une chromatographie liquide, le premier groupe d'échantillons de sang étant connu pour contenir de l'hémoglobine A1c, et étant connu pour contenir moins d'hémoglobine F qu'un rapport de teneur prédéterminé par rapport à l'hémoglobine totale, et la première équation de corrélation étant une équation de corrélation entre une valeur de pic d'hémoglobine A1c (40) et une valeur de pic composite incluant un pic d'hémoglobine A1a (11) et un pic d'hémoglobine A1b (12) ;

le calcul d'une valeur de pic d'hémoglobine F modifiée par soustraction d'une valeur de pic composite obtenue en appliquant, à la première équation de corrélation, une valeur de pic d'hémoglobine A1c (40) d'un chromatogramme obtenu en soumettant un échantillon de sang cible de mesure à une chromatographie liquide, d'une valeur de pic composite incluant un pic d'hémoglobine A1a (11) et un pic d'hémoglobine A1b (12) dans l'échantillon de sang cible de mesure ; et

la correction d'une valeur de pic d'hémoglobine F par ajout de la valeur de pic d'hémoglobine F modifiée à la valeur de pic d'hémoglobine F de l'échantillon de sang cible de mesure.

2. Procédé de mesure de l'hémoglobine F pour un échantillon de sang cible de mesure, le procédé comprenant :

la détermination préliminaire d'une première équation de corrélation à partir d'un chromatogramme obtenu en soumettant un premier groupe d'échantillons de sang à une chromatographie liquide, le premier groupe d'échantillons de sang étant connu pour contenir de l'hémoglobine A1c, et étant connu pour contenir moins d'hémoglobine F qu'un rapport de teneur prédéterminé par rapport à l'hémoglobine totale, et la première équation de corrélation étant une équation de corrélation entre une valeur de pic d'hémoglobine A1c (40) et une valeur de pic composite incluant un pic d'hémoglobine A1a (11) et un pic d'hémoglobine A1b (12) ;

l'obtention d'une valeur de pic composite par application d'une valeur de pic d'hémoglobine A1c (40) à la première équation de corrélation, la valeur de pic d'hémoglobine A1c (40) étant obtenue à partir d'un chromatogramme obtenu en soumettant un second groupe d'échantillons de sang connu pour contenir de l'hémoglobine A1c et de l'hémoglobine F à une chromatographie liquide ;

l'obtention d'une valeur de pic d'hémoglobine F modifiée estimée du second groupe d'échantillons de sang par soustraction de la valeur de pic composite d'une valeur de pic composite incluant un pic d'hémoglobine A1a (11) et un pic d'hémoglobine A1b (12) dans le second groupe d'échantillons de sang ;

la détermination préliminaire d'une deuxième équation de corrélation en utilisant une valeur de pic d'hémoglobine F et la valeur de pic d'hémoglobine F modifiée estimée du second groupe d'échantillons de sang, la deuxième équation de corrélation étant une équation de corrélation entre une valeur de pic d'hémoglobine F et une valeur de pic d'hémoglobine F modifiée ;

le calcul d'une valeur de pic d'hémoglobine F modifiée par application d'une valeur de pic d'hémoglobine F d'un chromatogramme obtenu en soumettant un échantillon de sang cible de mesure à une chromatographie liquide, à la deuxième équation de corrélation ; et

la correction d'une valeur de pic d'hémoglobine F par ajout de la valeur de pic d'hémoglobine F modifiée à la valeur de pic d'hémoglobine F de l'échantillon de sang cible de mesure.

3. Procédé de mesure de l'hémoglobine F pour un échantillon de sang cible de mesure, le procédé comprenant :

la détermination préliminaire d'une première équation de corrélation à partir d'un chromatogramme obtenu en soumettant un premier groupe d'échantillons de sang à une chromatographie liquide, le premier groupe d'échantillons de sang étant connu pour contenir de l'hémoglobine A1c, et étant connu pour contenir moins d'hémoglobine F qu'un rapport de teneur prédéterminé par rapport à l'hémoglobine totale, et la première équation de corrélation étant une équation de corrélation entre une valeur de pic d'hémoglobine A1c (40) et une valeur de pic composite incluant un pic d'hémoglobine A1a (11) et un pic d'hémoglobine A1b (12) ;

l'obtention d'une valeur de pic composite par application d'une valeur de pic d'hémoglobine A1c (40) à la première équation de corrélation, la valeur de pic d'hémoglobine A1c (40) étant obtenue à partir d'un chromatogramme obtenu en soumettant un second groupe d'échantillons de sang connu pour contenir de l'hémoglobine A1c et de l'hémoglobine F à une chromatographie liquide ;

l'obtention d'une valeur de pic d'hémoglobine F modifiée estimée du second groupe d'échantillons de sang par soustraction de la valeur de pic composite d'une valeur de pic composite incluant un pic d'hémoglobine A1a (11) et un pic d'hémoglobine A1b (12) dans le second groupe d'échantillons de sang ; et

la détermination préliminaire d'une deuxième équation de corrélation en utilisant une valeur de pic d'hémoglobine F et la valeur de pic d'hémoglobine F modifiée estimée du second groupe d'échantillons de sang, la deuxième équation de corrélation étant une équation de corrélation entre une valeur de pic d'hémoglobine F et une valeur de pic d'hémoglobine F modifiée ;

dans lequel, dans un cas dans lequel un chromatogramme obtenu en soumettant un échantillon de sang cible de mesure à une chromatographie liquide inclut un pic d'hémoglobine A1c (40), le procédé comprend en outre :

le calcul d'une valeur de pic d'hémoglobine F modifiée par soustraction d'une valeur de pic composite obtenue en appliquant une valeur de pic d'hémoglobine A1c (40) de l'échantillon de sang cible de mesure à la première équation de corrélation, d'une valeur de pic composite incluant un pic d'hémoglobine A1a (11) et un pic d'hémoglobine A1b (11) de l'échantillon de sang cible de mesure ; et

la correction d'une valeur de pic d'hémoglobine F par ajout de la valeur de pic d'hémoglobine F modifiée à la valeur de pic d'hémoglobine F de l'échantillon de sang cible de mesure, ou

dans lequel, dans un cas dans lequel un chromatogramme obtenu en soumettant un échantillon de sang cible de mesure à une chromatographie liquide n'inclut pas un pic d'hémoglobine A1c (40), le procédé comprend en outre :

le calcul d'une valeur de pic d'hémoglobine F modifiée par application d'une valeur de pic d'hémoglobine F de l'échantillon de sang cible de mesure à la deuxième équation de corrélation ; et

la correction d'une valeur de pic d'hémoglobine F par ajout de la valeur de pic d'hémoglobine F modifiée à la valeur de pic d'hémoglobine F de l'échantillon de sang cible de mesure.

4. Procédé de mesure de l'hémoglobine F pour l'échantillon de sang cible de mesure selon la revendication 2 ou la revendication 3, dans lequel :

une troisième équation de corrélation qui est une équation de corrélation entre une valeur de pic d'hémoglobine F et une somme de la valeur de pic d'hémoglobine F et de la valeur de pic d'hémoglobine F modifiée estimée dans le second groupe d'échantillons de sang est préliminairement déterminée au lieu de la deuxième équation de corrélation ; et

une valeur de pic d'hémoglobine F d'un chromatogramme obtenu en soumettant l'échantillon de sang cible de mesure à une chromatographie liquide est appliquée à la troisième équation de corrélation au lieu de la deuxième équation de corrélation, pour corriger la valeur de pic d'hémoglobine F.

5. Procédé de mesure de l'hémoglobine F pour l'échantillon de sang cible de mesure selon l'une quelconque de la revendication 1 à la revendication 4, dans lequel la chromatographie liquide est une chromatographie par échange de cations.

# FIG.1

# FIG.2

# FIG.3

## FIG.4

## FIG.5

FIG.6

FIG.7

# FIG.8

# FIG.9

**EP 4 249 905 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2014235023 A
- JP 2001228133 A
- JP 2021001804 A